# FASCICULE DE BREVET EUROPEEN

(11) **EP 0 522 639 B1**
(45) Date de publication et mention de la délivrance du brevet: **26.04.1995**
(21) Numéro de dépôt: 92201999.7
(22) Date de dépôt: 02.07.1992
(51) Int. Cl.: C07C 19/08, C07C 17/20

(54) **Procédé pour la préparation de 1-chloro-1,1,3,3,3-pentafluoropropane et de 1,1,1,3,3,3-hexafluoropropane**
Verfahren zur Herstellung von 1-Chlor-1,1,3,3,3-Pentafluorpropan und von 1,1,1,3,3,3-Hexafluorpropan
Process for the preparation of 1-chloro-1,1,3,3,3-pentafluoropropane and of 1,1,1,3,3,3-hexafluoropropane

(30) Priorité: 10.07.1991 BE 9100658
(43) Date de publication de la demande: 13.01.1993
(73) Titulaire: SOLVAY (Société Anonyme), B-1050 Bruxelles (BE)
(72) Inventeur: Pennetreau, Pascal, B-1310 La Hulpe (BE)
(74) Mandataire: Jacques, Philippe

(56) Documents cités:
- EP-A- 0 187 643
- WO-A-90/08754
- FR-A- 1 319 071
- CHEMICAL ABSTRACTS, vol. 113, no. 1, 2 Juillet 1990, Columbus, Ohio, US; abstract no. 5704U, page 555 ;

## Description

La présente invention concerne un procédé pour la préparation de 1-chloro-1,1,3,3,3-pentafluoropropane (HFA-235fa) et de 1,1,1,3,3,3-hexafluoropropane (HFA-236fa).

Elle concerne plus particulièrement un procédé de préparation de 1-chloro-1,1,3,3,3-pentafluoropropane et de 1,1,1,3,3,3-hexafluoropropane au départ de 1,1,1,3,3,3-hexachloropropane, en une étape.

Suite au problème de l'appauvrissement de la couche d'ozone et aux limitations de production et d'utilisation imposées en conséquence pour les chlorofluorocarbures entièrement halogénés (CFC), il existe aujourd'hui un intérêt accru pour les hydrocarbures chlorofluorés partiellement halogénés (HFA).

Il est notamment connu d'utiliser le 1-chloro-1,1,3,3,3-fluoropropane (demande de brevet EP-A-0394993 de DAIKIN) ou le 1,1,1,3,3,3-hexafluoropropane (demande de brevet JP-A-02 272086 de ASAHI GLASS) dans des mélanges caloporteurs et d'utiliser le 1-chloro-1,1,3,3,3-pentafluoropropane pour le gonflage de mousses (demande de brevet JP-A- 02 120335 de ASAHI GLASS).

Henne et al. (J.Am.Chem.Soc., 68, 1946, p.496) enseignent la préparation de 1-chloro-1,1,3,3,3-pentafluoropropane et de 1,1,1,3,3,3-hexafluoropropane au départ de 1,1,1-trichloro-3,3,3-trifluoropropane en présence de fluorure mercurique. Pour cette réaction, ils mettent en oeuvre comme produit de départ un composé ayant subi plusieurs étapes préalables de fluoration et de chloration.

Henne et al. (J. Am.Chem.Soc., 70, 1948, p.758) mentionnent par ailleurs la préparation de 1-chloro-1,1,3,3,3-pentafluoropropane par hydrofluoration de 1-chloro-1,3,3,3-tétrafluoropropène en présence de trifluorure de bore, réaction d'addition qui ne permet pas la préparation de 1-chloro-1,1,3,3,3-pentafluoropropane au départ de composés moins fluorés que le 1-chloro-1,3,3,3-tétrafluoropropène.

La présente invention a pour but de procurer un procédé permettant la préparation de 1-chloro-1,1,3,3,3-pentafluoropropane et de 1,1,1,3,3,3-hexafluoropropane avec une bonne sélectivité, au départ de 1,1,1,3,3,3-hexachloropropane, en une seule étape.

A cet effet, l'invention concerne un procédé pour la préparation de 1-chloro-1,1,3,3,3-pentafluoropropane et de 1,1,1,3,3,3-hexafluoropropane par réaction en phase liquide de 1,1,1,3,3,3-hexachloropropane avec du fluorure d'hydrogène, en présence d'un catalyseur.

Le 1,1,1,3,3,3-hexachloropropane mis en oeuvre au départ du procédé selon l'invention s'obtient avantageusement par réaction du chlorure de vinylidène avec le tétrachlorométhane.

Il est ainsi possible d'obtenir le 1-chloro-1,1,3,3,3-pentafluoropropane et le 1,1,1,3,3,3-hexafluoropropane en deux étapes, au départ de chlorure de vinylidène et de tétrachlorométhane, produits simples et largement disponibles.

Comme catalyseur de la réaction en phase liquide de 1,1,1,3,3,3-hexachloropropane avec du fluorure d'hydrogène, on peut mettre en oeuvre des catalyseurs d'hydrofluoration favorisant la substitution d'un atome de chlore par un atome de fluor. Parmi les catalyseurs utilisables, on peut citer les dérivés des métaux choisis parmi les métaux des groupes IIIa, IVa et b, Va et b, VIb du tableau périodique des éléments et leurs mélanges. On retient plus spécialement les dérivés de titane, de tantale, de molybdène, de bore, d'ètain et d'antimoine. De préférence, on met en oeuvre des dérivés d'étain ou d'antimoine. Les dérivés de l'antimoine conviennent particulièrement bien. Comme dérivés des métaux, on peut citer les sels et plus particulièrement les halogénures. De préférence, on choisit parmi les chlorures, les fluorures et les chlorofluorures. Des catalyseurs particulièrement préférés selon la présente invention sont les chlorures, les fluorures et les chlorofluorures d'étain et d'antimoine et leurs mélanges. Les chlorures conviennent particulièrement bien. Le pentachlorure d'antimoine s'est révélé particulièrement intéressant en ce qu'il résulte de son utilisation une sélectivité élevée en 1-chloro-1,1,3,3,3-pentafluoropropane et 1,1,1,3,3,3-hexafluoropropane.

On peut également utiliser un cocatalyseur.

La quantité de catalyseur mise en oeuvre dans le procédé peut varier dans de larges limites. Elle est généralement d'au moins 0,005 mole de catalyseur par mole de 1,1,1,3,3,3-hexachloropropane. Le plus souvent, elle ne dépasse pas 0,1 mole de catalyseur par mole de 1,1,1,3,3,3-hexachloropropane. De préférence, elle est d'au moins 0,02 mole de catalyseur par mole de 1,1,1,3,3,3-hexachloropropane. Il est également préférable qu'elle n'excède pas 0,06 mole de catalyseur par mole de 1,1,1,3,3,3-hexachloropropane.

Le rapport molaire entre le fluorure d'hydrogène et le 1,1,1,3,3,3-hexachloropropane mis en oeuvre est généralement d'au moins 4. Le plus souvent, ce rapport molaire ne dépasse pas 20. De préférence, on travaille avec un rapport molaire d'au moins 6. Il est également préférable que ce rapport molaire ne dépasse pas 17. Une excéllente sélectivité en 1-chloro-1,1,3,3,3-pentafluoropropane et en 1,1,1,3,3,3-hexafluoropropane est obtenue avec un rapport molaire d'au moins 8.

La température à laquelle s'effectue la réaction selon le procédé est généralement d'au moins 50°C. Le plus souvent, elle ne dépasse pas 150°C. De préférence, elle est d'au moins 100°C. Il est également préférable qu'elle ne dépasse pas 120°C.

La pression est choisie de manière à maintenir le milieu réactionnel sous forme liquide. Elle varie en fonction de la température du milieu réactionnel. Elle est généralement d'au moins 2 bar. Le plus souvent, elle ne dépasse pas 50 bar. La pression est de préférence d'au moins 10 bar. Il est également préférable qu'elle ne dépasse pas 40 bar. D'excellents résultats sont obtenus sans qu'il soit nécessaire de dépasser 30 bar.

De manière avantageuse on élimine en continu tout ou partie du chlorure d'hydrogène formé par la réaction. En général on élimine au moins 30% du chlorure d'hydrogène. Dans la mesure du possible on essaie d'éliminer la plus grande partie du chlorure d'hydrogène tout en veillant à maintenir dans le réacteur les réactifs et autres produits de réaction.

De manière surprenante on a constaté qu'il était possible de produire de grandes quantités de 1,1,1,3,3,3-hexafluoropropane et de 1-chloro-1,1,3,3,3-pentafluoropropane avec une bonne vitesse de réaction en éliminant ainsi au moins une partie du chlorure d'hydrogène formé.

Une technique avantageuse consiste à effectuer la réaction dans un réacteur à l'ébullition de manière à éliminer en continu le chlorure d'hydrogène formé. Le réacteur à l'ébullition est avantageusement surmonté d'une colonne de rectification afin de parfaire la séparation entre le chlorure d'hydrogène et les autres produits.

Dans une telle technique lorsqu'on souhaite produire essentiellement le 1,1,1,3,3,3-hexafluoropropane on peut de manière intéressante prélever une fraction riche en ce produit dans un des plateaux de la colonne. Cette fraction riche en 1,1,1,3,3,3-hexafluoropropane et contenant également du 1-chloro-1,1,3,3,3-pentafluoropropane, du fluorure d'hydrogène et de petites quantités d'autres produits peut si nécessaire être ensuite soumise à une décantation pour séparer une phase riche en fluorure d'hydrogène que l'on recycle au réacteur et une phase organique que l'on soumet à des distillations azéotropiques pour en séparer successivement un mélange azéotropique contenant du fluorure d'hydrogène que l'on renvoie au décanteur, du 1,1,1,3,3,3-hexafluoropropane qui est le produit souhaité et un mélange d'organiques moins fluorés que l'on recycle au réacteur.

Si on désire produire simultanément du 1-chloro-1,1,3,3,3-pentafluoropropane, on soumet le mélange d'organiques moins fluorés à une distillation pour le recueillir en tête de colonne avant de recycler les lourds au réacteur.

La description des autres façons de procéder qui suit concerne la production simultanée de 1,1,1,3,3,3-hexafluoropropane et 1-chloro-1,1,3,3,3, pentafluoropropane. Les techniques proposées peuvent être appliquées à une production exlusive de 1,1,1,3,3,3-hexafluoropropane; dans ce cas les différentes compositions débarrassées du 1,1,1,3,3,3-hexafluoropropane et contenant du 1-chloro-1,1,3,3,3-pentafluoropropane sont recyclées au réacteur. S'il s'agit d'un réacteur à l'ébullition on les recycle soit dans le bouilleur soit dans un des plateaux de la colonne. De manière avantageuse on introduit le produit recyclé dans un des plateaux de la moitié inférieure de la colonne.

Si on souhaite produire simultanément le 1-chloro-1,1,3,3,3-pentafluoropropane et le 1,1,1,3,3,3-hexafluoropropane, on peut aussi soumettre lá phase gazeuse en équilibre avec le mélange réactionnel à une distillation pour en éliminer le chlorure d'hydrogène.

Une façon de procéder consiste à effectuer la réaction à l'ébullition et à soumettre en continu la phase gazeuse obtenue par ébullition à une distillation pour en séparer le chlorure d'hydrogène en tête et en pied un liquide riche en 1,1,1,3,3,3-hexafluoropropane et contenant également du 1-chloro-1,1,3,3,3-pentafluoropropane et du fluorure d'hydrogène. Ce liquide peut être séparé en ses différents constituants. Le fluorure d'hydrogène et les organiques insuffisamment fluorés sont recyclés au réacteur. Des purges peuvent être prévues.

La séparation du liquide riche en 1,1,1,3,3,3-hexafluoropropane et contenant également du 1-chloro-1,1,3,3,3-pentafluoropropane et du fluorure d'hydrogène peut se faire par décantation éventuellement sous refroidissement de manière à en séparer une phase liquide riche en fluorure d'hydrogène que l'on recycle au réacteur et une phase organique que l'on soumet à des distillations organiques azéotropiques pour en séparer successivement un mélange azéotropique contenant du fluorure d'hydrogène que l'on renvoie au réacteur, du 1,1,1,3,3,3-hexafluoropropane, du 1-chloro-1,1,3,3,3-pentafluoropropane et un mélange d'organiques à recycler.

Selon une autre variante, le liquide riche en 1,1,1,3,3,3-hexafluoropropane et contenant également du 1-chloro-1,1,3,3,3-pentafluoropropane et du fluorure d'hydrogène peut être distillé pour séparer en tête de colonne un mélange riche en 1,1,1,3,3,3-hexafluoropropane et contenant du fluorure d'hydrogène d'un mélange riche en 1-chloro-1,1,3,3,3-pentafluoropropane et contenant du fluorure d'hydrogène ainsi que des produits plus lourds. Chacun de ces deux mélanges est indépendamment soumis à décantation sous refroidissement éventuel avec séparation de la phase riche en fluorure d'hydrogène que l'on recycle au réacteur. Le 1,1,1,3,3,3-hexafluoropropane recueilli peut si nécessaire faire l'objet d'une distillation azéotropique ultime. Le mélange riche en 1-chloro-1,1,3,3,3-pentafluoropropane et contenant des produits plus lourds mais débarrassé de l'essentiel du fluorure d'hydrogène est soumis à une série de distillations azéotropiques pour recueillir le 1-chloro-1,1,3,3,3-pentafluoropropane, les autres produits étant recyclés.

Une autre technique consiste à soumettre après refroidissement la phase gazeuse obtenue dans le réacteur à l'ébullition à une décantation pour obtenir une phase gazeuse riche en chlorure d'hydrogène, une fraction liquide riche en fluorure d'hydrogène que l'on recycle au réacteur et une fraction liquide contenant les organiques. Cette dernière est soumise à distillation pour obtenir en tête un produit riche en 1,1,1,3,3,3-hexafluoropropane dont on élimine les traces de fluorure d'hydrogène et de chlorure d'hydrogène par lavage à l'eau et en pied des organiques lourds dont on peut séparer le 1-chloro-1,1,3,3,3-pentafluoropropane par distillation. Les produits insuffisamments fluorés sont recyclés au réacteur.

La durée de la réaction nécessaire pour assurer une sélectivité optimale en 1-chloro-1,1,3,3,3-pentafluoropropane et en 1,1,1,3,3,3-hexafluoropropane est variable en fonction des conditions opératoires et sera évaluée dans chaque cas par voie expérimentale. L'évolution de la composition du milieu réactionnel peut notamment être suivie par le dosage par chromatographie en phase vapeur de prélèvements réalisés à intervalles réguliers.

Le procédé selon l'invention peut être réalisé dans tout réacteur réalisé dans des matériaux résistant à la pression et au fluorure d'hydrogène. On utilise souvent des réacteurs réalisés en acier, en acier inoxydable ou en alliages tels que ceux connus sous les marques MONEL, INCONEL ou HASTELLOY. On peut également utiliser des réacteurs munis d'un revêtement en un métal ou alliage inerte, ou recouverts d'une couche d'une résine inerte dans les conditions de réaction, notamment des résines fluorées.

Comme déjà mentionné, le 1,1,1,3,3,3-hexachloropropane mis en oeuvre au départ du procédé selon l'invention peut être obtenu par télomérisation du chlorure de vinylidène avec le tétrachlorométhane et notamment de la manière décrite par Belbachir et al. (Makromol. Chem., 185, 1984, 1583-1595).

Cette réaction est avantageusement effectuée en présence d'un catalyseur du type des peroxydes ou des sels de fer ou de cuivre et, en particulier, du chlorure cuivreux, lequel offre une excellente sélectivité en 1,1,1,3,3,3-hexachloropropane produit.

Ladite réaction est par ailleurs avantageusement réalisée en présence d'un solvant polaire donneur tel que le diméthylsulfoxyde ou l'acétonitrile, l'acétonitrile offrant d'excellents résultats.

Avant sa mise en oeuvre dans le procédé selon l'invention, le 1,1,1,3,3,3-hexachloropropane peut être purifié, notamment par distillation sous pression réduite.

Les exemples qui suivent sont donnés dans le but d'illustrer l'invention mais ne sont nullement limitatifs.

### Exemple 1

### a. Préparation du 1,1,1,3,3,3-hexachloropropane

On utilise un autoclave de 1 l recouvert d'une résine fluorocarbonée TEFLON® , équipé d'un agitateur, d'une sonde de température et d'un tube plongeant permettant d'effectuer des prélèvements en cours d'essais.

On introduit dans cet autoclave 1,8 mole de chlorure de vinylidène, 3,6 mole de tétrachlorométhane, 0,018 mole de chlorure cuivreux et 3,6 mole d'acétonitrile.

Le milieu réactionnel est agité et chauffé à 140°C pendant 13 heures. La pression autogène atteint 5,9 bar.

On refroidit alors l'autoclave jusqu'à la température ambiante et on le vide.

L'analyse révèle que le taux de transformation du chlorure de vinylidène est alors de 95 % en moles et la sélectivité en 1,1,1,3,3,3-hexachloropropane est de 87 % molaires par rapport au chlorure de vinylidène transformé.

Le 1,1,1,3,3,3-hexachloropropane obtenu est purifié par distillation sous pression réduite.

### b. Hydrofluoration du 1,1,1,3,3,3-hexachloropropane

On utilise un autoclave de 0,5 l en acier inox, équipé d'un agitateur, d'un système de régulation de pression, d'une sonde de température et d'un tube plongeant permettant d'effectuer des prélèvements en cours d'essais.

On fait le vide dans ce réacteur et on le refroidit à - 20°C.

On y introduit successivement 0,4 mole de 1,1,1,3,3,3-hexachloropropane, 0,02 mole de pentachlorure d'antimoine et 3,2 mole de fluorure d'hydrogène.

Le milieu réactionnel est ensuite progressivement chauffé jusque 110°C et la pression est régulée à 25 bar.

Après 3,5 heures, le taux de transformation du 1,1,1,3,3,3-hexachloropropane est de 98 % et la sélectivité globale en 1-chloro-1,1,3,3,3-pentafluoropropane et en 1,1,1,3,3,3-hexafluoropropane est de plus de 40 % molaires par rapport au 1,1,1,3,3,3-hexachloropropane transformé.

Après 6 heures, le taux de transformation du 1,1,1,3,3,3-hexachloropropane est de 100 % et la sélectivité globale en 1-chloro-1,1,3,3,3-pentafluoropropane et en 1,1,1,3,3,3-hexafluoropropane est de plus de 45 % molaires par rapport au 1,1,1,3,3,3-hexachloropropane transformé.

En fin de réaction, le milieu est refroidi et transvasé dans une ampoule à décanter en polyéthylène remplie d'eau, de manière à séparer le fluorure d'hydrogène n'ayant pas réagi. La phase organique est décantée et le 1-chloro-1,1,3,3,3-pentafluoropropane et le 1,1,1,3,3,3-hexafluoropropane sont purifiés par distillation.

### Exemple 2

Le mode opératoire est analogue à celui de l'exemple 1.

On introduit successivement dans le réacteur 0,9 mole de 1,1,1,3,3,3-hexachloropropane, 0,045 mole de tétrachlorure d'étain et 8,1 mole de fluorure d'hydrogène.

Le milieu réactionnel est ensuite progressivement chauffé jusque 110°C et la pression est régulée à 18 bar en éliminant une partie du HCl formé.

Après 46 heures, le taux de transformation du 1,1,1,3,3,3-hexachloropropane est de 100 % et la sélectivité globale en 1-chloro-1,1,3,3,3-pentafluoropropane et en 1,1,1,3,3,3-hexafluoropropane est de 32 % molaires par rapport au 1,1,1,3,3,3-hexachloropropane transformé.

Après 50 heures, la sélectivité globale en 1-chloro-1,1,3,3,3-pentafluoropropane et en 1,1,1,3,3,3-hexafluoropropane atteint 39 % molaires.

## Revendications

1. Procédé pour la préparation de 1-chloro-1,1,3,3,3-pentafluoropropane et de 1,1,1,3,3,3-hexafluoropropane, caractérisé en ce que l'on fait réagir en phase liquide du 1,1,1,3,3,3-hexachloropropane avec du fluorure d'hydrogène, en présence d'un catalyseur.

2. Procédé selon la revendication 1, caractérisé en ce que le catalyseur est choisi parmi les dérivés des métaux des groupes IIIa, IVa et b, Va et b, VIb du tableau périodique des éléments et leurs mélanges.

3. Procédé selon la revendication 2, caractérisé en ce que les dérivés des métaux sont choisis parmi les chlorures, les fluorures et les chlorofluorures.

4. Procédé selon la revendication 1, caractérisé en ce que le catalyseur est choisi parmi les chlorures, les fluorures et les chlorofluorures d'étain et d'antimoine et leurs mélanges.

5. Procédé selon la revendication 4, caractérisé en ce que le catalyseur est le pentachlorure d'antimoine.

6. Procédé selon la revendication 1, caractérisé en ce que le catalyseur est mis en oeuvre à raison de 0,005 à 0,1 mode de catalyseur par mode de 1,1,1,3,3,3-hexachloropropane.

7. Procédé selon la revendication 1, caractérisé en ce que le rapport molaire entre le fluorure d'hydrogène et le 1,1,1,3,3,3-hexachloropropane mis en oeuvre est d'au moins 4 et ne dépasse pas 20.

8. Procédé selon la revendication 1, caractérisé en ce que la température à laquelle la réaction est réalisée est d'au moins 50°C et ne dépasse pas 150°C.

9. Procédé selon la revendication 1, caractérisé en ce que la pression sous laquelle la réaction est réalisée est d'au moins 2 bar et ne dépasse pas 50 bar.

10. Procédé selon la revendication 1, caractérisé en ce que le 1,1,1,3,3,3-hexachloropropane mis en oeuvre est obtenu par réaction du chlorure de vinylidène avec le tétrachlorométhane en présence de chlorure cuivreux et d'acétonitrile.

## Claims

1. Process for preparing 1-chloro-1,1,3,3,3-pentafluoropropane and 1,1,1,3,3,3-hexafluoropropane, characterised in that 1,1,1,3,3,3-hexachloropropane is reacted in the liquid phase with hydrogen fluoride, in the presence of a catalyst.

2. Process according to Claim 1, characterised in that the catalyst is chosen from derivatives of metals of groups IIIa, IVa and b, Va and b and VIb of the Periodic Table of the elements and their mixtures.

3. Process according to Claim 2, characterised in that the derivatives of the metals are chosen from chlorides, fluorides and chlorofluorides.

4. Process according to Claim 1, characterised in that the catalyst is chosen from tin and antimony chlorides, fluorides and chlorofluorides and their mixtures.

5. Process according to Claim 4, characterised in that the catalyst is antimony pentachloride.

6. Process according to Claim 1, characterised in that the catalyst is used in the ratio of 0.005 to 0.1 mol of catalyst per mol of 1,1,1,3,3,3-hexachloropropane.

7. Process according to Claim 1, characterised in that the mole ratio of hydrogen fluoride to the 1,1,1,3,3,3-hexachloropropane used is at least 4 and does not exceed 20.

8. Process according to Claim 1, characterised in that the temperature at which the reaction is carried out is at least 50°C and does not exceed 150°C.

9. Process according to Claim 1, characterised in that the pressure at which the reaction is carried out is at least 2 bar and does not exceed 50 bar.

10. Process according to Claim 1, characterised in that the 1,1,1,3,3,3-hexachloropropane used is obtained by reacting vinylidene chloride wish carbon tetrachloride in the presence of cuprous chloride and acetonitrile.

## Patentansprüche

1. Verfahren zur Herstellung von 1-Chlor-1,1,3,3,3-pentafluorpropan und von 1,1,1,3,3,3-Hexafluorpropan, dadurch gekennzeichnet, daß man in flüssiger Phase 1,1,1,3,3,3-Hexachlorpropan mit Fluorwasserstoff in Gegenwart eines Katalysators umsetzt.

2. Verfahren gemäß Anspruch 1, dadurch gekennzeichnet, daß der Katalysator unter den Derivaten der Metalle der Gruppen IIIa, IVa und b, Va und b, VIb des Periodensystems der Elemente und ihren Gemischen ausgewählt ist.

3. Verfahren gemäß Anspruch 2, dadurch gekennzeichnet, daß die Derivate der Metalle unter den Chloriden, den Fluoriden und den Chlorfluoriden ausgewählt sind.

4. Verfahren gemäß Anspruch 1, dadurch gekennzeichnet, daß der Katalysator unter den Chloriden den Fluoriden und den Chlorfluoriden des Zinn und des Antimon und ihren Gemischen ausgewählt ist.

5. Verfahren gemäß Anspruch 4, dadurch gekennzeichnet, daß der Katalysator Antimonpentachlorid ist.

6. Verfahren gemäß Anspruch 1, dadurch gekennzeichnet, daß der Katalysator in einer Menge von 0,005 bis 0,1 Mol Katalysator pro Mol 1,1,1,3,3,3-Hexachlorpropan eingesetzt wird.

7. Verfahren gemäß Anspruch 1, dadurch gekennzeichnet, daß das eingesetzte Molverhältnis zwischen dem Fluorwasserstoff und dem 1,1,1,3,3,3-Hexachlorpropan wenigstens 4 beträgt und 20 nicht übersteigt.

8. Verfahren gemäß Anspruch 1, dadurch gekennzeichnet, daß die Temperatur, bei der die Reaktion durchgeführt wird, wenigstens 50 °C beträgt und 150 °C nicht übersteigt.

9. Verfahren gemäß Anspruch 1, dadurch gekennzeichnet, daß der Druck, unter dem die Reaktion durchgeführt wird, wenigstens 2 bar beträgt und 50 bar nicht übersteigt.

10. Verfahren gemäß Anspruch 1, dadurch gekennzeichnet, daß das eingesetzte 1,1,1,3,3,3-Hexachlorpropan durch Reaktion von Vinylidenchlorid mit Tetrachlormethan in Gegenwart von Kupfer(I)-chlorid und Acetonitril erhalten wird.
